(19) Europäisches Patentamt / European Patent Office / Office européen des brevets

(11) **EP 2 061 832 B1**

(12) **FASCICULE DE BREVET EUROPEEN**

(45) Date de publication et mention de la délivrance du brevet:
**04.10.2017 Bulletin 2017/40**

(21) Numéro de dépôt: **07823452.3**

(22) Date de dépôt: **28.08.2007**

(51) Int Cl.:
*C08J 9/00* (2006.01)    *C08J 9/30* (2006.01)
*A61L 15/42* (2006.01)    *A47L 13/16* (2006.01)
*A61F 13/53* (2006.01)    *A61L 15/22* (2006.01)
*C08J 5/04* (2006.01)    *D04H 1/68* (2012.01)

(86) Numéro de dépôt international:
**PCT/FR2007/001405**

(87) Numéro de publication internationale:
**WO 2008/025898 (06.03.2008 Gazette 2008/10)**

(54) **MATERIAU ABSORBANT ET SON PROCEDE DE PREPARATION**

ABSORBIERENDE SUBSTANZ UND HERSTELLUNGSVERFAHREN DAFÜR

ABSORBANT MATERIAL AND ITS PREPARATION METHOD

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **29.08.2006 FR 0607576**

(43) Date de publication de la demande:
**27.05.2009 Bulletin 2009/22**

(73) Titulaire: **MAPA**
**92705 Colombes Cedex (FR)**

(72) Inventeurs:
• **GAROIS, Nicolas**
  **45200 Amilly (FR)**
• **SONNTAG, Philippe**
  **77850 Hericy (FR)**
• **CARNIOL, Natacha**
  **45200 Amilly (FR)**

(74) Mandataire: **Le Roux, Martine**
**Cabinet Beau de Loménie**
**158, rue de l'Université**
**75340 Paris Cedex 07 (FR)**

(56) Documents cités:
**US-A1- 2001 024 716    US-B1- 6 346 557**

EP 2 061 832 B1

**Description**

L'invention se rapporte à un nouveau matériau alvéolaire, à un procédé pour sa préparation et à ses utilisations, notamment comme matériau absorbant, et en particulier pour la fabrication d'éponges et autres produits à usage ménager.

Dans le domaine de l'entretien ménager, on utilise principalement des éponges végétales, à base de cellulose régénérée, et des éponges synthétiques qui sont le plus souvent constituées par des mousses de polyuréthanne à cellules ouvertes.

Si les éponges à base de cellulose régénérée présentent, d'une manière générale, des qualités très satisfaisantes, tant en termes de capacités d'absorption et de rétention d'eau, d'aptitude à essuyer, de souplesse, de résilience, de résistance mécanique et de résistance à l'eau, aux détergents et à la chaleur, leur fabrication pose, par contre, des problèmes majeurs.

En effet, ces éponges sont fabriquées par des procédés qui consistent à transformer, dans un premier temps, de la cellulose en une pâte de viscose, transformation qui est réalisée par sodation de la cellulose, dissolution de l'alcali-cellulose ainsi formée par du sulfure de carbone et traitement du xanthate de cellulose résultant par de la lessive de soude. Puis, après incorporation dans la pâte de viscose ainsi obtenue, de fibres de renfort (chanvre, lin, coton, ...), de colorants et de cristaux de sulfate de soude, et mise en forme par moulage ou boudinage, l'ensemble est soumis à un chauffage qui permet de solidifier la viscose, de la régénérer en cellulose par évaporation du sulfure de carbone et de faire fondre les cristaux de sulfate de soude qui, en s'éliminant, laissent à leur place une multitude d'alvéoles.

Aussi, la mise en oeuvre de ces procédés à une échelle industrielle, compte tenu de la nature de la nature très corrosive et toxique des produits qu'ils utilisent, nécessite des installations très spécifiques et très coûteuses, tant en termes d'investissements que de coûts de fonctionnement, est hautement polluante en dépit des équipements de dépollution que ces installations comportent et des mesures qui sont prises pour limiter les nuisances sur l'environnement et ce, pour des rendements de production relativement faibles.

Les éponges en mousses de polyuréthanne sont obtenues par des procédés de fabrication nettement moins contraignants, qui sont basés sur une réaction de condensation entre un polyol et un polyisocyanate dans une phase aqueuse, mais elles présentent l'inconvénient d'avoir un caractère relativement hydrophobe qui se traduit par des propriétés de mouillabilité, de rétention d'eau et d'essuyage très insuffisantes et ce, en dépit des nombreux traitements qui ont été proposés dans l'art antérieur pour rendre les mousses de polyuréthanne plus hydrophiles.

Il a, par ailleurs, été proposé dans US-A-4,559,243, de réaliser des structures spongieuses se présentant sous la forme de feuilles de quelques mm d'épaisseur, en déposant une mousse constituée par un mélange d'un latex et de fibres hydrophiles du type fibres de cellulose, de viscose ou encore d'alcool polyvinylique sur un support tel qu'un tissu, un non tissé ou une feuille plastique, puis en soumettant l'ensemble à des opérations de chauffage de manière à obtenir la coagulation de la mousse et sa stabilisation en une structure à cellules ouvertes par séchage et réticulation. Si la fabrication de ces structures spongieuses est dépourvue, comme celle des éponges en mousses de polyuréthanne, des inconvénients des procédés de fabrication des éponges végétales, il s'avère toutefois que ces structures présentent un faible pouvoir absorbant qui limite considérablement leur intérêt.

On connaît, par le document WO99/09877, des matières spongieuses comprenant un mélange de fibres de cellulose et d'au moins un élastomère qui présente une structure alvéolaire formée par des alvéoles dont la taille est comprise entre 0,01 et 10 mm, une densité comprise entre 0,03 et 0,1, une capacité d'absorption d'eau au moins égale à 750%, et une capacité de rétention d'eau après un essorage manuel inférieure à 100%. Ces matières spongieuses sont préparées par un procédé consistant à mélanger des fibres de cellulose et un élastomère sous forme de latex, incorporer dans le mélange un agent apte à conférer au produit une structure alvéolaire (glace, agent moussant), mettre en forme ce mélange, et appliquer un traitement apte à assurer la réticulation du produit. Si la capacité d'absorption d'eau de ces éponges est généralement jugée satisfaisante, en revanche leur pouvoir d'essuyage peut encore être amélioré.

En outre, les éponges obtenues par ce procédé, tout comme les autres éponges de l'art antérieur, présentent à l'utilisateur une sensation rêche au toucher. Cette sensation désagréable est un inconvénient généralement reconnu des éponges à base de cellulose. S'il est accepté, faute de solution plus satisfaisante, pour les éponges et autres produits ménagers, il constitue un obstacle au développement de nouvelles applications pour les produits à structure poreuse à base de cellulose.

Certains produits commercialisés actuellement ont été conçu pour résoudre ce problème de sensation rêche au toucher : ce sont des structures spongieuses se présentant sous la forme de feuilles en matériau tissé ou non tissé de quelques mm d'épaisseur, auquel on a imprimé des micro perforations à l'aide d'une pointe appliquée perpendiculairement au plan de la feuille. De tels produits sont décrits, par exemple, dans la Demande de Brevet US2005/0276956A1. Toutefois, ces produits, s'ils ont un toucher agréable, ont une capacité d'absorption très limitée et un pouvoir d'essuyage faible. En outre, le procédé de micro perforation ne s'applique qu'à des structures de faible épaisseur et ne permet pas d'accéder à une structure alvéolaire tridimensionnelle.

La Demanderesse s'est, en conséquence, fixé pour but de fournir des matériaux alvéolaires, à base de fibre de polymère hydrophile, en particulier à base de cellulose, qui présentent un toucher doux et agréable pour l'utilisateur, qui puissent être produits en toutes formes et notamment en articles de toutes épaisseurs. Elle a en outre cherché à obtenir des produits qui présentent toutes les qualités requises pour un usage ménager et, notamment, une capacité à absorber un grand volume d'eau et à retenir l'eau ainsi absorbée aussi longtemps que l'on ne cherche pas à l'éliminer de manière active, une aptitude à libérer toutefois cette eau sous l'effet d'un essorage manuel, un pouvoir d'essuyage élevé, et dont la fabrication soit simple à mettre en oeuvre, ne nécessite pas d'investissements industriels importants, n'utilise ni produits corrosifs, ni produits toxiques, soit dénuée de retentissement sur l'environnement et se caractérise par des rendements de production économiquement intéressants.

Ce but est atteint, selon la présente Invention, par une matière alvéolaire comprenant un mélange de fibres de polymère hydrophile, en particulier de cellulose et d'au moins un élastomère, caractérisée en ce qu'elle présente une structure alvéolaire formée par des alvéoles dont la taille est comprise entre 0,2 $\mu$m et 10 mm, au moins 1% des alvéoles, en volume par rapport au volume alvéolaire total, ayant une taille comprise entre 0,2 $\mu$m et 10$\mu$m.

Avantageusement, le matériau de l'invention répond à au moins l'une, et de préférence plusieurs, des caractéristiques suivantes :

Il est doté d'une densité comprise entre 0,01 et 0,1, préférentiellement entre 0,02 et 0,06.

Il a une capacité d'absorption d'eau au moins égale à 800%.

Il a une capacité de rétention d'eau après un essorage manuel inférieure à 100%.

Au sens de la présente Invention, on entend par "capacité d'absorption d'eau", le rapport exprimé en pourcentage entre la masse d'eau susceptible d'être absorbée par le matériau alvéolaire lorsque celle-ci est entièrement immergée dans un volume d'eau et la masse sèche de ce matériau alvéolaire, et par "capacité de rétention d'eau après un essorage manuel", le rapport, également exprimé en pourcentage, entre la masse d'eau retenue dans le matériau alvéolaire après que celui-ci ait été essoré manuellement et la masse sèche dudit matériau alvéolaire.

Les fibres de polymère hydrophile sont préférentiellement des fibres de cellulose, mais elles peuvent être également choisies parmi les fibres de dérivés de la cellulose, comme par exemple l'acétate de cellulose, l'hydroxypropyl cellulose, la viscose, ou d'autres polymères naturels ou synthétiques sous forme de fibres comme des polysaccharides, du polyméthylène méthacrylate. Les fibres de cellulose utiles selon l'Invention sont toutes les fibres de cellulose naturelle telles que les fibres cellulosiques du bois ou fibres papetières (fibres de résineux ou de feuillus, blanchies ou non), les fibres de coton, de lin, de chanvre, de jute, de sisal ou encore les fibres régénérées de chiffons.

Elles peuvent, par ailleurs, être des fibres longues (c'est-à-dire des fibres mesurant plus d'1 cm de long), des fibres courtes (de longueur inférieure à 3 mm) ou des fibres de longueur intermédiaire (entre 3 mm et 1 cm de long) ou encore être composées d'un mélange de fibres de différentes longueurs. Ainsi, par exemple, d'excellents résultats ont été obtenus en utilisant soit des fibres cellulosiques longues, préparées par découpage de feuilles de linters de coton en lambeaux de quelques cm de côté, seules ou en association avec des fibres cellulosiques courtes telles que celles commercialisées sous le nom commercial ARBOCELL® par la Société RETTENMAIER & SOHNE et qui mesurent environ 900 $\mu$m de longueur, soit des fibres cellulosiques de longueur intermédiaire, également préparées par découpage de feuilles de linters de coton, mais en lambeaux de longueur sensiblement comprise entre 8 mm et 1 cm.

Par ailleurs, quelle que soit leur longueur, les fibres de polymères hydrophiles, en particulier les fibres cellulosiques utilisables dans l'Invention peuvent avantageusement avoir été soumises préalablement à un traitement propre à favoriser leur enchevêtrement au sein de l'élastomère et, partant, leur adhésion vis-à-vis de cet élastomère. Un tel traitement peut consister, par exemple, en une fibrillation, c'est-à-dire un brassage mécanique ayant pour effet de libérer des fibrilles à la surface des fibres leur permettant de s'accrocher les unes aux autres, ou en une exposition aux ultraviolets qui, en induisant la formation de sites réactifs à la surface des fibres, autorise un accrochage chimique de ces fibres. A titre d'exemple de fibres cellulosiques ayant subi une fibrillation disponibles commercialement, on peut citer les fibres commercialisées sous le nom commercial LYOCELL® par la Société COURTAULDS CHEMICALS.

Autrement dit, les fibres de polymères hydrophiles utiles dans l'invention peuvent être un mélange de fibres de différentes natures, de différentes longueurs et toutes ces fibres ou seulement une partie de ces fibres peuvent avoir subi un traitement.

De préférence, le mélange sera un mélange de fibres courtes et de fibres longues.

L'élastomère utile selon l'Invention peut, quant à lui, être choisi parmi de très nombreux élastomères pour autant que ces élastomères soient compatibles avec le polymère hydrophile, et notamment la cellulose, et ne présentent donc pas un caractère hydrophobe prononcé.

Ainsi, l'élastomère sera avantageusement sélectionné parmi les caoutchoucs polybutadiène, les copolymères de butadiène-styrène, les copolymères butadiène-acrylonitrile, les caoutchoucs nitrile, les caoutchoucs nitrile-butadiène (NBR), les copolymères et terpolymères d'éthylène et de propylène, les copolymères séquencés styrène-butadiène ou styrène-isoprène, les copolymères séquencés styrène-éthylène-butylène-styrène, les élastomères thermoplastiques dérivés des polyoléfines (tels que le SANTOPRENE® de la Société AES ou le VEGAPRENE® de la Société HUTCHINSON), les copolymères d'octène et d'éthylène (tels que ceux commercialisés par la Société DU PONT DOW sous le nom commercial ENGAGE®), les copolymères d'éthylacrylate et autres acrylates comme les terpolymères d'acrylate, d'éthylène et d'acide acrylique (tels que ceux commercialisés par les Sociétés DU PONT DE NEMOURS et EXXON, respectivement sous les références VAMAC® et ATX® 325) ou les terpolymères d'acrylate, d'acrylonitrile et de styrène (comme le SUNIGUM® de la Société GOODYEAR), les polychloroprènes, les polyéthylènes chlorés, et leurs mélanges.

Par ailleurs, pour ce qui concerne les élastomères polyoléfiniques précités et, notamment, les caoutchoucs polybutadiène, butadiène-styrène et butadiène-acrylonitrile, l'utilisation des dérivés carboxylés de ces élastomères s'est révélée particulièrement avantageuse en raison de leur aptitude à former, par pontages ioniques entre les fonctions carboxyle en présence de métaux divalents ou trivalents tels que le zinc, le calcium ou l'aluminium, un réseau contribuant à conférer au matériau alvéolaire de l'invention une cohésion satisfaisante.

Conformément à l'Invention, le matériau alvéolaire peut comprendre, en plus des fibres de polymère hydrophile (en particulier des fibres de cellulose), des fibres synthétiques propres à jouer un rôle de renfort au sein de l'élastomère et permettant soit d'augmenter encore la cohésion de ce matériau alvéolaire et, partant, sa résistance mécanique lorsque cela s'avère nécessaire, soit de réduire la quantité d'élastomère nécessaire à l'obtention d'une cohésion appropriée et d'abaisser ainsi le prix de revient dudit matériau.

A titre d'exemples de fibres synthétiques susceptibles de convenir, on peut citer les fibres de polyamide, les fibres de polyester, les fibres de polyéthylène, les fibres de polypropylène, les fibres de polyacrylonitrile et les fibres d'alcool polyvinylique, étant entendu que, quelle que soit la nature chimique des fibres choisies, on utilisera de préférence des fibres présentant à la fois suffisamment de ténacité pour qu'elles puissent jouer leur rôle de fibres de renfort, et suffisamment de souplesse pour éviter qu'elles ne rigidifient le matériau alvéolaire finalement obtenu. En tout état de cause, lorsque de telles fibres de renfort sont présentes dans le matériau alvéolaire, elles représentent avantageusement au plus 20% et, de préférence, entre 5 et 15% en masse de la masse totale des fibres présentes dans ce matériau.

Le matériau alvéolaire conforme à l'Invention peut, également, de façon avantageuse, comprendre un ou plusieurs polymères propres à servir d'agents d'interface entre les fibres de polymère hydrophile, notamment les fibres cellulosiques (et, éventuellement, les fibres synthétiques) et l'élastomère, et à favoriser ainsi leur adhésion mutuelle. Pour ce faire, ce ou ces polymères auront, de préférence, une nature plus hydrophile que celle de l'élastomère.

A titre d'exemples de polymères susceptibles d'être utilisés, on peut citer les alcools polyvinyliques (ELVANOL®de la Société DU PONT DE NEMOURS, GOHSENOL® de la Société NIPPON GOSHEI, ...), les résines mélamine-formaldéhyde (CYREZ 963 E de la Société CYTEC, RESIMENE s 3521 de la Société MONSANTO, ...), les colles vinyliques ou colles à bois ou encore les polyuréthannes. Lorsque de tels polymères sont présents dans le matériau alvéolaire, ils peuvent représenter jusqu'à 35 parties en masse pour 100 parties en masse de l'élastomère.

Le matériau alvéolaire peut, de plus, comprendre un ou plusieurs adjuvants convenablement choisis, en fonction des propriétés que l'on souhaite lui donner, parmi les adjuvants classiquement employés dans l'industrie des polymères. Elle peut, ainsi, contenir des charges claires du type silices, carbonates, argiles, craies ou kaolins, des plastifiants, des colorants ou pigments, des stabilisants tels que des antioxydants, des agents anti-ultraviolets, des antiozonants, des fongicides, des bactéricides, des parfums microencapsulés ainsi que des agents de mise en oeuvre propres à faciliter sa fabrication tels que des agents épaississants, des agents tensioactifs, des agents de coagulation du latex ou encore des agents de réticulation, comme i1 sera explicité ci-après.

Selon une première disposition préférée du matériau alvéolaire conforme à l'Invention, le rapport entre la masse totale des fibres (fibres hydrophiles, en particulier fibres de cellulose et, éventuellement, fibres synthétiques) et la masse d'élastomère présentes dans ce matériau est compris entre 2 et 0,2 et, de préférence, entre 1,5 et 0,3.

Le matériau alvéolaire peut présenter des alvéoles présentant toutes la même taille ou sensiblement la même taille. Toutefois, on préfère que la taille de ces alvéoles soit hétérogène et se répartisse selon une large distribution de manière à former un réseau tridimensionnel de microcavités et de macro cavités au sein du matériau alvéolaire, apte à favoriser la capacité d'absorption d'eau de ce matériau ainsi que sa capacité de rétention d'eau avant essorage (de manière à ce que l'eau ne s'égoutte pas sous l'effet de la gravité), et à lui conférer, de plus, la souplesse nécessaire pour rendre son essorage facile. Par rapport aux matériaux plats micro perforés, l'existence de ce réseau d'alvéoles tridimensionnel permet d'obtenir de bien meilleures capacités d'essuyage et de rétention d'eau.

Selon la présente invention, le matériau alvéolaire présente une structure formée par des alvéoles dont la taille est comprise entre 0,2 $\mu$m et 10 mm, au moins 1% des alvéoles, en volume par rapport au volume alvéolaire total, ayant une taille comprise entre 0,2 $\mu$m et 10 $\mu$m.

Préférablement, pour augmenter encore sa capacité d'absorption d'eau, le matériau alvéolaire de l'invention est de plus doté d'au moins 10%, en volume par rapport au volume alvéolaire total, d'alvéoles ayant une taille comprise entre 10 $\mu$m et 50$\mu$m.

Par rapport aux matériaux alvéolaires de l'art antérieur, le matériau de l'invention présente la particularité d'être doté d'alvéoles de très petite taille qui lui confèrent une capacité de rétention d'eau améliorée et un toucher plus agréable, notamment plus doux. Par rapport aux matériaux plats micro perforés, qui ont également un toucher agréable, les matériaux alvéolaires de l'invention présentent l'avantage de pouvoir être fabriqués suivant des formats et des dimensions très variés.

Ainsi, c'est la présence à la fois d'alvéoles de très petites tailles et d'alvéoles de plus grandes tailles qui confère ses qualités au matériau alvéolaire de l'invention.

Avantageusement le matériau alvéolaire conforme à l'Invention présente une densité comprise entre 0,03 et 0,05 et une capacité d'absorption d'eau comprise entre 1000 et 1600%. De préférence il présente un taux d'essorage inférieur à 90%. Avantageusement, il présente une capacité d'essuyage supérieure ou égale à 65%, avantageusement supérieure ou égale à 70%.

La capacité d'essuyage d'un matériau est définie par la quantité d'eau qu'il a absorbée après essuyage d'une surface mouillée.

Selon encore une autre disposition avantageuse du matériau alvéolaire conforme à l'Invention, il présente, de plus, une résistance à la traction au moins égale à 0,1 MPa.

Ainsi, le matériau alvéolaire conforme à l'Invention présente de nombreux avantages : outre d'avoir un haut pouvoir absorbant, il est capable de retenir l'eau absorbée tant que l'on ne cherche pas à l'éliminer de manière active, tout en la libérant sous l'effet d'un essorage manuel. Il présente, par ailleurs, un pouvoir d'essuyage élevé. Il possède, de plus, une souplesse qui rend sa manipulation aisée ainsi qu'une résilience qui lui permet de reprendre sa forme initiale après chaque essorage. Il présente, en outre, des propriétés mécaniques et, notamment, de résistance à la contrainte extrêmement satisfaisantes. Enfin, des tests d'analyse sensorielle ont montré la supériorité du matériau alvéolaire de l'invention en matière de douceur au toucher.

Le matériau alvéolaire conforme à l'Invention est, de ce fait, particulièrement bien adapté à entrer dans la constitution d'éponges et, notamment, d'éponges destinées à la toilette ou au nettoyage de surfaces. Pour ce faire, elle présente une épaisseur comprise, de préférence, entre 1 et 15 cm, de manière particulièrement préférée, entre 1,5 et 10 cm et de manière encore plus préférée, entre 2 et 5 cm pour faciliter la prise en main de ces éponges.

Le matériau alvéolaire peut également être employé pour la fabrication d'éponges plates, d'une épaisseur allant généralement de 1mm à 5 mm. Dans ce cas, le procédé de fabrication du matériau alvéolaire est adapté pour que la taille maximale des alvéoles soit préférentiellement inférieure ou égale à 0,5mm. Les éponges plates fabriquées à partir du matériau alvéolaire de l'invention ont une douceur comparable ou meilleure par rapport à celle des éponges en tissé ou en non-tissé micro perforé de l'art antérieur, en revanche, leurs capacités d'absorption et d'essuyage sont très supérieures.

Les qualités au toucher de ce matériau alvéolaire permettent également d'envisager son utilisation dans la fabrication de produits destinés à un contact corporel, comme par exemple les produits de soin ou d'hygiène corporelle ou l'habillement. Parmi les produits destinés à l'hygiène corporelle, on peut citer les éponges destinées au bain, en substitution des éponges naturelles ; les éponges de soin ou de démaquillage, qui peuvent être commercialisées sous forme sèche ou pré-imprégnées d'un produit de soin ou de démaquillage ; les couches pour bébés et les articles d'hygiène féminine ; les pansements ; les articles destinés à absorber la sueur, notamment à l'usage des sportifs, tels que bandeaux, carrés de toilette... Parmi les produits d'habillement, on peut citer les combinaisons en mousse destinées notamment à la pratique des sports nautiques.

En fonction des additifs que l'on incorpore aux matériaux alvéolaires de l'invention, des applications de ceux-ci peuvent être envisagées dans d'autres domaines techniques, comme par exemple dans le domaine de l'imprimerie ou du bâtiment.

La présente Invention a, également, pour objet un procédé de préparation d'un matériau alvéolaire tel que précédemment défini, qui est caractérisé en ce qu'il comprend :

a) préparation d'une dispersion aqueuse, dénommée $A_1$, de fibres d'au moins un polymère hydrophile préférentiellement de cellulose,
b) préparation d'une composition, dénommée $B_1$, d'au moins un élastomère sous la forme d'un latex,
c) ajout d'un agent de coagulation du latex à la dispersion $A_1$,
d) mélange de la dispersion $A_1$ obtenue à l'étape c) et de la composition $B_1$,
e) élimination du surplus d'agent de coagulation ;
f) optionnellement préparation d'une dispersion aqueuse, dénommée $A_2$, de fibres d'au moins un polymère hydrophile, la dispersion $A_2$ étant identique ou différente de la dispersion aqueuse $A_1$, et incorporation de cette dispersion aqueuse $A_2$ dans le mélange obtenu à l'issue de l'étape e),
g) préparation d'une composition, dénommée $B_2$, d'au moins une élastomère sous la forme d'un latex, la composition $B_2$ étant identique ou différente de la composition $B_1$, et incorporation de cette composition $B_2$ dans le mélange obtenu à l'étape e) ou à l'étape f), si une étape f) est mise en oeuvre,
h) application d'un traitement apte à conférer une structure alvéolaire au mélange obtenu à l'étape g) ;
i) la mise en forme du mélange obtenu à l'étape h),
j) application au produit obtenu à l'étape i) d'un traitement apte à assurer la coagulation et la réticulation dudit produit, et

k) séchage du produit obtenu à l'étape j).

Ainsi, dans un premier mode de mise en oeuvre préféré du procédé de l'invention, le procédé ne comporte pas l'étape optionnelle f) et la composition $B_2$ est identique à la composition $B_1$.

Dans un second mode de mise en oeuvre préféré du procédé de l'invention, le procédé ne comporte pas l'étape optionnelle f), mais la composition $B_2$ est différente de la composition $B_1$.

Dans un troisième mode de réalisation préféré du procédé de l'invention, le procédé comporte l'étape optionnelle f) et la dispersion $A_1$ est une dispersion de fibres longues, la dispersion $A_2$ est une dispersion de fibres courtes et la composition $B_2$ est identique à la composition $B_1$.

Dans un quatrième mode de mise en oeuvre préféré du procédé de l'invention, le procédé comprend l'étape optionnelle f), la dispersion $A_1$ est une dispersion différente de la dispersion $A_2$, et la composition $B_2$ est différente de la composition $B_1$.

Mais, dans un cinquième mode de mise en oeuvre préféré du procédé de l'invention, le procédé comporte l'étape optionnelle f), et la dispersion $A_1$ est identique à la dispersion $A_2$, toutes deux comprennent un mélange de fibres courtes, et de fibres longues et éventuellement de fibres intermédiaires et la composition $B_2$ est identique à la composition $B_1$.

Dans un sixième mode de mise en oeuvre du procédé de l'invention, le procédé comporte l'étape optionnelle f), la dispersion $A_1$ est identique à la dispersion $A_2$, et la composition $B_2$ est différente de la composition $B_1$.

La dispersion $A_1$, comme l'éventuelle dispersion $A_2$, peut contenir un mélange de fibres de différentes natures, de différentes longueurs, l'essentiel étant qu'une partie au moins de ces fibres soit traitée par un agent de coagulation de latex et mélangée à une composition de latex pour coagulation.

Dans tous les cas, la dispersion des fibres de polymère hydrophile, et notamment de cellulose, dans la phase aqueuse peut être réalisée en introduisant ces fibres dans un mélangeur préalablement rempli d'un volume d'eau convenablement choisi et sous une agitation mécanique appropriée (qui, d'une manière générale, sera d'autant plus vigoureuse que les fibres de cellulose seront plus longues), et en maintenant cette agitation jusqu'à l'obtention d'une pâte homogène. Quel que soit le type de mélangeur (turbodisperseur, mélangeur planétaire, agitateur muni d'une pâle défloculeuse, ...) dans lequel on effectue cette dispersion, il est avantageux que ce mélangeur soit équipé d'un système permettant d'éviter ou, à tout le moins, de limiter l'échauffement de la dispersion, comme par exemple un système de réfrigération des parois.

Il convient de remarquer que, dans le cas où l'on souhaite que le matériau alvéolaire de l'invention contienne, outre des fibres de polymère hydrophile, des fibres synthétiques, il est tout à fait possible, conformément à ce premier mode de mise en oeuvre préféré, d'ajouter ces fibres synthétiques aux fibres de polymère hydrophile, par exemple en les dispersant conjointement avec ces dernières dans la phase aqueuse.

De manière similaire, si l'on désire utiliser un ou plusieurs polymères aptes à servir d'agents d'interface entre les fibres et l'élastomère et/ou un ou plusieurs adjuvants, ceux-ci peuvent être incorporés soit dans la dispersion de fibres de polymère hydrophile, soit dans le latex, soit encore dans leur mélange tel qu'obtenu à l'étape d) et/ou à l'étape f).

Dans le mode de mise en oeuvre préféré du procédé de l'invention, l'agent de coagulation du latex est un acide. Dans ce cas l'étape c) d'ajout d'un agent de coagulation du latex à la dispersion $A_1$ est une acidification de la dispersion $A_1$, jusqu'à obtention d'un pH compris entre 1 et 4.

Cet acide peut être une solution aqueuse d'un acide faible, qu'il soit de nature organique ou minérale. On peut citer par exemple l'acide acétique, l'acide formique, l'acide oxalique, l'acide succinique, l'acide maléique, l'acide fumarique, l'acide citrique, l'acide ascorbique. Selon une variante de l'invention, l'étape c) peut être réalisée simultanément à l'étape a) en dispersant les fibres de polymère hydrophile tout en procédant à l'acidification.

Dans ce mode de mise en oeuvre préféré du procédé de l'invention, l'étape e) d'élimination du surplus d'agent de coagulation est alors une étape de neutralisation du mélange obtenu à l'étape d).

Mais la coagulation du latex peut être obtenue de manière connue dans l'art par d'autres méthodes, dont la méthode de coagulation par électrolyte.

Dans cette méthode de coagulation par électrolyte, la composition de latex à coaguler est plongée dans une solution d'électrolyte, généralement une solution de chlorure de calcium ou de nitrate de calcium, puis retirée et lavée pour éliminer les sels.

On peut envisager une coagulation thermique du latex sur les fibres préalablement chauffées.

Dans le mode de mise en oeuvre préféré de l'invention, dans lequel la coagulation du latex est obtenue par acidification, l'étape d'élimination des sels par lavage est évitée et remplacée par une simple neutralisation.

Le latex d'élastomère contient, outre l'élastomère lui-même, et de façon connue, des tensio-actifs appropriés, cationiques ou anioniques, pour stabiliser l'élastomère, un ou plusieurs agents plastifiants.

Ainsi, l'incorporation de la composition $B_1$ de latex se fait par mélange dans les mêmes conditions que décrit ci-dessus pour la dispersion des fibres de polymère hydrophile. Lorsque la dispersion aqueuse de fibres contenant l'agent de coagulation de latex est mélangée avec la composition B$_1$ du latex d'élastomère, ce mélange provoque la coagulation du latex.

Ensuite, le latex coagulé doit être réticulé. Soit le latex est autoréticulant, auquel cas la présence d'un agent de réticulation n'est pas nécessaire, soit le latex n'est pas autoréticulant et un agent de réticulation est nécessaire. Les agents de réticulation du latex sont bien connus de l'homme de l'art et comprennent les composés soufrés, les péroxydes, etc.

L'agent de réticulation, lorsque nécessaire, est ajouté au latex dans des proportions comprises, de préférence, entre 0,05 et 0,5 part en masse pour 100 parts de masse sèche de l'élastomère présent dans ce latex

Par rapport aux procédés de l'art antérieur, le procédé de l'invention se distingue notamment en ce que la coagulation d'une partie du latex est provoquée avant la formation de la structure alvéolaire, ce qui permet d'obtenir une répartition bimodale ou trimodale de la tailles des alvéoles, c'est-à-dire d'une part des alvéoles de très petite taille et, d'autre part des alvéoles de taille plus élevée.

Préférentiellement, la composition B$_1$ représente de 10 à 80% en poids par rapport au poids total de latex introduit (composition B$_1$ + composition B$_2$). Par conséquent, la composition B$_2$ représente de 20 à 90% en poids par rapport au poids total de latex introduit.

Dans le mode de mise en oeuvre préféré du procédé de l'invention dans lequel l'agent de coagulation obtenu à l'étape c) est un acide, l'élimination du surplus d'acide est effectué par traitement du mélange à l'aide d'une solution aqueuse basique, comme par exemple une solution aqueuse de soude, qui est ajoutée jusqu'à obtention d'un pH neutre.

L'incorporation de la composition B$_2$ de latex se fait par mélange dans les mêmes conditions que décrit ci-dessus pour la dispersion des fibres de polymère hydrophile.

Le traitement apte à conférer une structure alvéolaire au matériau peut être constitué par l'injection d'un gaz qui, en étant introduit dans le mélange de fibres de polymère hydrophile/latex/latex coagulé, va générer au sein de ce mélange une multitude de bulles et le transformer en une mousse, ou par battage. La mousse est ensuite solidifiée, ce qui assure un maintien des bulles qu'elle renferme. On obtient ainsi une structure alvéolaire, interconnectée et se caractérisant avantageusement par une distribution de la taille des alvéoles comportant des élément alvéolaires de l'ordre du micron.

De manière préférée, le gaz est de l'air et il est introduit dans le mélange dispersion de fibres de polymère hydrophile/latex/latex coagulé en soumettant ce mélange pendant quelques minutes à une agitation mécanique vigoureuse, avantageusement comprise entre 800 et 1200 tr/min, et ce, par exemple dans un turbodisperseur qui, là également, peut être muni d'un système propre à éviter ou, à tout le moins, limiter l'échauffement du mélange tel qu'un système de réfrigération des parois. Il est toutefois possible d'utiliser un gaz autre que l'air, comme par exemple un gaz inerte, pour réaliser cette opération de moussage.

Dans la mesure où la vitesse à laquelle est effectuée l'agitation mécanique du mélange dispersion de fibres de cellulose/latex/latex coagulé, et la durée de cette agitation règlent la densité et la taille des alvéoles du matériau alvéolaire finalement obtenu - à savoir que ces dernières seront d'autant plus faibles que l'agitation aura été plus vigoureuse et plus longue-, la vitesse et la durée de cette agitation seront donc avantageusement choisies en fonction des propriétés que l'on souhaite conférer au matériau alvéolaire de l'invention.

Selon l'invention, le produit à structure alvéolaire obtenu à l'issue de l'étape h) est mis en forme en fonction de l'utilisation ultérieure qui est prévue. La mise en forme peut comporter une étape d'extrusion, par exemple sous forme de bande, ou une étape de moulage.

Par exemple, selon une variante du procédé de l'invention, l'élastomère étant un élastomère réticulable, la mise en forme du mélange fibres de cellulose/élastomère/élastomère coagulé est réalisée par extrusion à une température comprise entre 60 et 80°C, puis le produit extrudé est chauffé à une température comprise entre 120 et 180°C, directement à la sortie de l'extrudeuse, par exemple par passage dans un tunnel à micro-ondes ou dans un tube de vapeur, pour obtenir son expansion et sa réticulation.

Selon une autre variante de ce procédé, l'élastomère étant un élastomère thermoplastique, la mise en forme du mélange fibres de cellulose/élastomère/élastomère coagulé est réalisée par extrusion à une température comprise entre 140 et 180°C et l'expansion du produit extrudé s'effectue spontanément à sa sortie de la filière.

Selon encore une autre variante de ce procédé, l'élastomère étant un élastomère réticulable, la mise en forme du mélange fibres de cellulose/élastomère/élastomère coagulé est réalisée par un calandrage suivi d'un moulage par compression, lequel est effectué à une température comprise entre 120 et 150°C et permet une réticulation partielle du produit moulé. Après démoulage, ce produit est chauffé à une température comprise entre 150 et 200°C, par exemple au moyen d'une étuve ou d'un autoclave à air chaud, pour obtenir son expansion et achever sa réticulation.

Selon encore une autre variante du procédé de l'invention, qui s'applique aussi bien dans le cas où l'élastomère est un élastomère réticulable que dans celui où il est thermoplastique, la mise en forme du mélange fibres de cellulose/élastomère est réalisée par le remplissage partiel d'un moule par injection ou par transfert, puis par l'expansion dudit mélange et, éventuellement, sa réticulation simultanée au sein du moule pour obtenir le remplissage total de ce dernier. Lorsque l'élastomère est un élastomère réticulable, le moule est préalablement chauffé, par exemple à une température comprise entre 150 et 200°C.

La solidification de la mousse, c'est-à-dire la coagulation et la réticulation du latex, est obtenue par une augmentation de la température de la mousse - c'est-à-dire en pratique d'un chauffage de cette dernière.

Avantageusement, la coagulation et réticulation du latex est réalisée en plaçant le produit sous forme de mousse à une température au moins égale à 25°C et, de préférence supérieure à 35°C, par exemple dans un tunnel à micro-ondes ou à infrarouges, un tube de vapeur, un autoclave en vapeur vive ou à air chaud, une étuve à air ventilé ou à air chaud, un four à haute fréquence, et en maintenant cette mousse à cette température suffisamment de temps pour obtenir sa gélification, soit en pratique pendant une durée comprise entre 1 et 5 heures selon l'épaisseur de la mousse et la nature du latex, entre autres et la réticulation complète du latex.

Conformément à l'Invention, la coagulation et la réticulation du latex est suivie d'une opération de chauffage du produit obtenu destinée à sécher et à compléter, si besoin est, la réticulation du latex. Cette opération de chauffage est réalisée en soumettant ledit produit, de préférence, à une température comprise entre 100 et 200°C - en utilisant là également un dispositif de chauffage du type tunnel à micro-ondes ou à infrarouges, tube de vapeur, autoclave en vapeur vive ou à air chaud, étuve à air ventilé ou à air chaud, four à haute fréquence, ou successivement plusieurs de ces dispositifs -, et en le maintenant à cette température pendant une durée comprise entre 1 et 5 heures selon les cas.

En pratique, il est possible et même avantageux de réaliser la coagulation, la réticulation et le séchage du produit en une seule étape et dans un seul dispositif de chauffage, en plaçant directement le produit sous forme de mousse dans ce dispositif préchauffé à la température choisie pour le séchage et la réticulation, la coagulation s'effectuant alors au cours de la montée en température de la mousse.

Conformément au procédé de l'Invention, celui-ci comprend, de plus, l'incorporation dans la dispersion de fibres de cellulose, le latex ou leur mélange selon le cas :

- d'un agent tensioactif propre à favoriser la transformation du mélange dispersion de fibres de cellulose/latex, en une mousse ; à titre d'exemples d'agents tensioactifs qui se sont révélés particulièrement bien convenir à la mise en oeuvre du procédé conforme à l'Invention, on peut citer les sulfosuccinates tels que ceux commercialisés par la Société CYTEC sous le nom commercial AEROSOL®; lorsqu'un tel agent tensioactif est utilisé, il est, de préférence, ajouté au latex à l'étape b), avant que ce dernier ne soit mélangé avec la dispersion de fibres de polymère hydrophile et ce, selon des proportions comprises entre 2 et 6 parts en masse pour 100 parts de masse sèche de l'élastomère présente dans ce latex ;

- d'un agent propre à stabiliser la mousse, une fois celle-ci formée ; un tel agent peut, notamment, être un agent épaississant tel qu'un éther ou un ester de cellulose (hydroxyéthylcellulose, hydroxypropyl méthylcellulose, ...) ; par ailleurs, cet agent est avantageusement incorporé dans la dispersion de fibres de polymère hydrophile, en particulier de cellulose, selon des proportions comprises entre 0,5 et 4 parts en masse de la masse sèche de l'élastomère présente dans le latex.

A titre d'exemple, d'excellents résultats ont été obtenus en préparant, conformément au procédé de l'invention, des matériaux alvéolaires présentant un rapport entre la masse sèche des fibres de polymère hydrophile (préférentiellement cellulose) et la masse sèche de l'élastomère d'environ 0,5, en mélangeant une dispersion de fibres de cellulose présentant une concentration en fibres comprise entre 8 et 15% avec un latex contenant une teneur en élastomère sec d'environ 55% dans des proportions permettant d'obtenir, en tenant compte des adjuvants qui leur sont ajoutés (système de réticulation et, éventuellement, charges, agents tensioactifs, agents épaississants, agents de coagulation, ...), un rapport entre la masse de matière sèche et la masse d'eau présentes dans ce mélange de l'ordre de 0,3.

Quel que soit le mode de mise en oeuvre du procédé conforme à l'Invention, ce procédé comprend, dès lors qu'il fait appel à l'utilisation d'un élastomère réticulable, l'incorporation d'un système de réticulation convenablement choisi en fonction de cet élastomère et pouvant comprendre, outre un agent de réticulation proprement dit (soufre, peroxydes), des promoteurs et des accélérateurs de réticulation, au cours de la préparation du mélange fibres de polymère hydrophile/élastomère, notamment étapes a), b), d), ou f).

De manière similaire, ce procédé comprend, dès lors qu'il utilise, en tant qu'agent d'interface entre les fibres cellulosiques (et, éventuellement, les fibres synthétiques) et l'élastomère, un polymère dont la réticulation nécessite la présence d'un système de réticulation spécifique - ce qui est par exemple le cas de l'alcool polyvinylique - l'addition d'un tel système de réticulation, lequel peut, là aussi, comprendre, non seulement un agent de réticulation proprement dit, mais également des promoteurs et des accélérateurs de réticulation.

Par ailleurs, quel que soit le mode de mise en oeuvre du procédé conforme à l'Invention, celui-ci comprend, de plus, la découpe du matériau alvéolaire obtenu aux dimensions et aux formes (blocs, plaques, feuilles, ...) appropriées aux usages auxquels il est destiné.

L'invention a pour objet tout article comprenant un matériau alvéolaire selon la présente invention.

En particulier, la présente Invention a pour objet des éponges, caractérisées en ce qu'elles comprennent un matériau alvéolaire tel que précédemment défini.

Ces éponges, qui peuvent être aussi bien destinées à la toilette qu'au nettoyage de surfaces, présentent une épaisseur comprise, de préférence, entre 1 et 15 cm, de manière particulièrement préférée, entre 1,5 et 10 cm et de manière encore plus préférée, entre 2 et 5 cm pour en faciliter la prise en main. Il peut aussi s'agir d'éponges plates, d'une épaisseur allant généralement de 1mm à 5 mm.

La présente Invention a, en outre, pour objets des articles de ménage tels que balais et raclettes pour l'entretien des surfaces (sols, murs, glaces, vitres, ...) comprenant un matériau alvéolaire selon l'invention.

La présente invention a encore pour objet les articles d'hygiène corporelle comprenant le matériau alvéolaire selon l'invention : les éponges destinées au bain ; les éponges de soin ou de démaquillage ; les couches pour bébés et les articles d'hygiène féminine ; les pansements ; les articles destinés à absorber la sueur.

La présente Invention sera mieux comprise à l'aide du complément de description qui suit et qui se réfère à des exemples de réalisation de matières spongieuses conformes à l'Invention et de démonstration de leurs propriétés et aux figures annexées dans lesquelles :

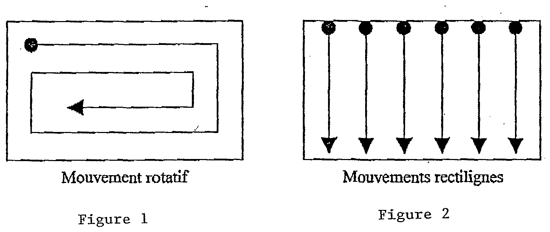

- la figure 1 représente le mouvement rotatif à effectuer pour réaliser le test de mesure de la capacité d'essuyage de l'éponge obtenue à l'exemple 1,
- la figure 2 représente les mouvements rectilignes à effectuer pour réaliser le test de mesure de la capacité d'essuyage de l'éponge obtenue à l'exemple 1.

Il va de soi, toutefois, que ces exemples sont donnés uniquement à titre d'illustrations de l'objet de l'Invention dont ils ne constituent en aucune manière une limitation.

**Exemple** 1 : **Fabrication d'une éponge selon l'invention**

On prépare une éponge à partir des constituants suivants :

| Composant | | Pourcentage de matière active (1) | Masses à doser (grammes) |
|---|---|---|---|
| Mélange Latex | Latex NBR | 55 | 1047,6 |
| | Tétramethylthiuram disulfide | 50 | 23,0 |
| | Zinc dibenzyldithiocarbamate | 45 | 25,6 |
| | Zinc 2-mercaptobenzothiazole | 50 | 23,0 |
| | TiO2 | 50 | 345,7 |
| | Colorant jaune | 19 | 3,0 |
| | Colorant blond | 11,9 | 24,2 |
| | **TOTAL mélange latex** | | **1492,2** |
| Mélange fibre | Linters de coton | 11,5 | 576,2 |
| | Eau | | 4434,0 |
| | Méthylhydroxypropylcellulose | 100 | 40,3 |
| | Hydroxyéthylcellulose | 100 | 17,3 |
| | Acide acétique | 100 | 144,0 |
| | **TOTAL** | | **6704,1** |
| Agent neutralisant | Hydroxyde de sodium | 50 | 195,9 |

(1) en poids par rapport au poids d'eau dans la matière première

Mode opératoire

On prépare le mélange latex en ajoutant les ingrédients suivant l'ordre de la formule.

On homogénéise le mélange latex.

On divise la quantité de mélange latex préparée en deux : mélange 1 et mélange 2 et on laisse chacun sous agitation.

On place les linters de coton, la méthylhydroxypropylcellulose, l'hydroxyéthylcellulose et l'eau dans le mélangeur (mélangeur à palles d'une capacité de 50 litres) pour le délitage des fibres : durée 5 minutes à 1050 tours/min. La proportion de ces deux agents rhéologiques peut varier sensiblement afin d'obtenir un diamètre moyen des cellules plus ou moins important tout en conservant le toucher doux du produit final.

On verse ensuite dans le mélangeur l'acide acétique puis on homogénéise 30 secondes à 600 tours/min.

On verse ensuite le mélange 1 puis on homogénéise pendant une durée de 30 secondes à 600 tours/min.

On ajoute alors l'agent neutralisant et l'on homogénéise 30 secondes à 300 tours/min.

On verse ensuite le mélange 2 puis on homogénéise 30 secondes à 600 tours/min.

On recueille la matière obtenue et on l'introduit dans la trémie du foisonneur continu (pompe : débit de 5 à 45 litres/heure, tête de mélangeage : capacité de 0,3 litres avec ergots carrés de 150x5 mm) pour mousser la matière.

On met la matière moussée dans des moules.

On sèche les moules à 140°C puis on les vulcanise à 160°C.

On démoule et on découpe des morceaux de la taille d'une éponge.

**Exemple 2 : Mesures des propriétés des matériaux alvéolaires de l'invention :**

Les propriétés des matériaux alvéolaires conformes à l'Invention ont été appréciées en déterminant :

- leur densité,
- leur capacité d'absorption d'eau,
- leur capacité de rétention d'eau après un essorage manuel,
- leur résistance à la traction,
- leur pouvoir d'essuyage, et
- la taille des alvéoles.

**Méthodes de mesures utilisées**

1) La densité a été déterminée en établissant le rapport (d) entre la masse volumique de ces matières spongieuses et la masse volumique de l'eau.

2) La capacité d'absorption d'eau a été déterminée en pesant les matières spongieuses lorsqu'elles sont parfaitement sèches et après immersion dans un volume d'eau, puis en établissant le rapport (A) selon la formule :

$$A = \frac{\text{masse après immersion dans l'eau - masse sèche}}{\text{masse sèche}} \times 100$$

tandis que la capacité de rétention d'eau après un essorage manuel a été déterminée en pesant ces mêmes des matériaux alvéolaires après un essorage manuel vigoureux et en établissant le rapport (R) selon la formule :

$$R = \frac{\text{masse après immersion dans l'eau - masse après essorage manuel}}{\text{masse sèche}} \times 100$$

3) La résistance à la traction a été, elle, déterminée en soumettant des éprouvettes mesurant entre 5 et 6 cm de longueur, 2,5 et 3,5 cm de largeur et entre 1,5 et 2,5 cm d'épaisseur et préparées par découpage des matériaux alvéolaires à tester, à une traction au moyen d'un dynamomètre électronique réglé à 300 mm/mn jusqu'à l'obtention d'une rupture.

4) Le pouvoir d'essuyage a été apprécié par l'existence ou non de traces d'eau sur une surface préalablement mouillée après essuyage de cette surface par lesdits matériaux alvéolaires, suivant la méthode suivante :

**Procédure du test d'essuyage :**

Mesure de la capacité d'essuyage des éponges :

I. Principe :

La Capacité d'essuyage (C) d'une éponge est quantifiée par la mesure de la quantité d'eau absorbée par l'éponge, après essuyage d'une surface mouillée.

II Matériel:

- Eau à 20 ± 2°C,
- Une balance de précision (au mg près),
- Une éponge de forme parallélépipédique de dimensions 100 mm x 100 mm x 20 mm,

- Un bécher plastique (volume = 51),
- Un miroir de format 54 cm x 39 cm,
- Une micropipette de 5 ml,
- De l'eau distillée,
- Un récurant hydrophobe de forme parallélépipédique de dimensions 100 mm x 100 mm x 5 mm,
- Une machine à laver le linge,
- Une chronomètre,
- De l'acétone,
- Du papier absorbant.

III Mode opératoire :

- Laver l'éponge à tester avec la machine à laver le linge au programme suivant :
- > Lavage (durée : 30 min, température de l'eau : 10°C)
- > Vidange (pas de pause avant vidange, durée de vidange : 20 s),
- > Essorage (durée : 20 s, vitesse finale : 200 trs/min)
- A la fin du programme, si en pressant l'éponge, de la mousse apparaît, relaver l'éponge au même programme. Répéter cette opération jusqu'à disparition de la mousse.
- Plonger l'éponge dans un bécher d'eau et presser l'éponge afin de la gorger d'eau.
- Essorer l'éponge avec la machine à laver le linge au programme suivant :
- > durée de l'essorage : 2 min, vitesse finale d'essorage ; 1000 trs/min.
- Pendant le temps de l'essorage, dégraisser le miroir avec du papier essuie-tout imbibé d'acétone ;
- Retirer l'éponge de la machine à laver le linge et poser la verticalement sur une surface sèche ;
- Laisser la porte de la machine à laver le linge grand ouvert.
- Tarer le récurant.
- Verser le long du miroir, 3 ml d'eau distillée à l'aide de la micropipette (soit 3 g d'eau).
- Répartir cette eau de façon uniforme à l'aide du récurant.
- Secouer le récurant au dessus du miroir pour éliminer les gouttes éventuellement retenues.
- Peser le récurant. Soit M' sa masse en g. Cette masse est soustraite aux 3 grammes d'au versée sur le miroir pour connaître la masse d'eau déposée sur la surface. Soit M1 cette masse en g.
- Déclencher le chronomètre ;
- Essuyer le miroir avec l'éponge préalablement tarée. Le geste doit d'abord être un mouvement rotatif de manière à ne passer qu'une seule fois partout (durée : 10 s). Puis un mouvement rectiligne avec aller simple (avec le même côté de l'éponge incliné à 45° environ), de manière à ne passer qu'une seule fois partout (durée : 8 s).
- Arrêter le chronomètre (la durée indiquée doit être de 18s $\pm$ 1s).
- Peser l'éponge. Soit M2 la masse en g (ne pas attendre la stabilisation de la balance pour prendre la valeur car l'eau s'évapore rapidement de l'éponge).
- Essuyer le miroir avec du papier absorbant.
- Dégraisser le miroir avec du papier essuie-tout imbibé d'acétone.
- La Capacité d'essuyage de l'éponge est exprimée en % par : C = 100 x M2/M1.
- Répéter les opérations d'essuyage en essorant l'éponge avec la machine à laver le linge au programme décrit précédemment, afin d'obtenir au final trois valeurs de C.
- Faites la moyenne des trois valeurs de C.
- La Capacité d'essuyage de l'éponge est égale à la moyenne calculée à $\pm$ 5 % près.

5) La taille des alvéoles a été déduite d'images MEB (Microscopie Electronique à Balayage) prises à 4 échelles différentes. On mesure le diamètre d des alvéoles sur les quatre séries d'images.

**Exemple 3 : Résultats des mesures des propriétés de l'éponge obtenue à l'exemple 1.**

On obtient une densité autour de 0,04 (pour le produit final). Cette densité est obtenue après réglage des paramètres du foisonneur tels que la vitesse de pompe, la vitesse de tête et l'injection d'air. Plus la densité du produit est faible, plus le toucher est doux.

Les contraintes maxi à la rupture sont de l'ordre de 0,1 MPa (dans le sens de la longueur et de la largeur) pour les produits obtenus.

L'absorption d'eau est d'environ 1300%.

La capacité d'essuyage est autour de 75%.

La mouillabilité est de 4 secondes.

Le taux d'essorage est de 85% environ.

La répartition de taille des alvéoles est la suivantes :

| | |
|---|---|
| $d > 1000 \ \mu m$ : | 77% |
| $1000 \ \mu m > d > 100 \ \mu m$ : | 17% |
| $100 \ \mu m > d > 10 \ \mu m$ : | 4% |
| $10 \ \mu m > d > 0,2 \ \mu m$: | 2% |

A titre de comparaison, une éponge réalisée par le procédé décrit dans le document WO99/09877, testée dans les mêmes conditions à une capacité d'essuyage de l'ordre de 65%.

## Revendications

1. Procédé de préparation d'un matériau alvéolaire comprenant un mélange de fibres de polymère hydrophile et d'au moins un élastomère, **caractérisé en ce qu'**il comprend :

   a) la préparation d'une dispersion aqueuse, dénommée $A_1$, de fibres d'au moins un polymère hydrophile, préférentiellement de cellulose,
   b) la préparation d'une composition, dénommée $B_1$, d'au moins un élastomère sous forme d'un latex,
   c) l'ajout d'un agent de coagulation du latex dans la dispersion $A_1$, cette étape c) pouvant être réalisée simultanément à l'étape a),
   d) le mélange de la dispersion $A_1$ obtenue à l'étape c) et de la composition $B_1$ ; ledit mélange provoquant la coagulation du latex,
   e) l'élimination du surplus d'agent de coagulation introduit à l'étape c) ;
   f) optionnellement, la préparation d'une dispersion aqueuse, dénommée $A_2$, de fibres d'au moins un polymère hydrophile, la dispersion $A_2$ étant identique ou différente de la dispersion aqueuse $A_1$, et l'incorporation de cette dispersion aqueuse $A_2$ dans le mélange obtenu à l'issue de l'étape e),
   g) la préparation d'une composition, dénommée $B_2$, d'au moins un élastomère sous la forme d'un latex, la composition $B_2$ étant identique ou différente de la composition $B_1$, et l'incorporation de cette composition $B_2$ dans le mélange obtenu à l'étape e) ou à l'étape f), si une étape f) est mise en oeuvre,
   h) l'injection d'un gaz dans le mélange - dispersion de fibres d'au moins un polymère hydrophile/latex/latex coagulé - obtenu à l'étape g) ou le battage dudit mélange, pour conférer audit mélange une structure alvéolaire,
   i) la mise en forme, notamment par extrusion ou par moulage, du mélange obtenu à l'étape h),
   j) le chauffage du produit obtenu à l'étape i) pour la coagulation et la réticulation dudit produit, et
   k) le séchage du produit obtenu à l'étape j), les étapes j) et k) pouvant être réalisées en une seule étape et dans un seul dispositif de chauffage.

2. Procédé selon la revendication 1, **caractérisé en ce que** lesdites fibres d'au moins un polymère hydrophile ont été soumises à une fibrillation ou exposées aux ultraviolets.

3. Procédé selon la revendication 1 ou 2, **caractérisé en ce qu'**à l'étape c), l'agent de coagulation est un acide qui est ajouté à la dispersion $A_1$ en une quantité permettant d'obtenir un pH entre 1 et 4, et **en ce que** l'étape e) est une étape de neutralisation du mélange obtenu à l'issue de l'étape d).

4. Procédé selon l'une quelconque des revendications 1 à 3, **caractérisé en ce que** $B_1$ représente de 10 à 80 % en poids par rapport au poids total de latex introduit et $B_2$ représente de 20 à 90 %, en poids par rapport au poids total de latex introduit.

5. Procédé selon l'une quelconque des revendications 1 à 4, **caractérisé en ce que** le gaz injecté à l'étape h), qui consiste de préférence en l'air, est introduit dans le mélange dispersion de fibres d'au moins un polymère hydrophile/latex/latex coagulé en soumettant ce mélange pendant quelques minutes à une agitation mécanique.

6. Procédé selon l'une quelconque des revendications 1 à 5, **caractérisé en ce que** l'étape j) est réalisée en plaçant le produit obtenu à l'étape h) à une température au moins égale à 25°C dans un tunnel à micro-ondes ou à infrarouges, un tube de vapeur, un autoclave en vapeur vive ou à air chaud, une étuve à air ventilé ou à air chaud, un four à haute fréquence, et en maintenant ce produit pendant une durée comprise entre 1 et 5 heures.

**7.** Procédé selon l'une quelconque des revendications 1 à 6, **caractérisé en ce que**, à l'étape k), le produit est soumis à une opération de chauffage en soumettant ledit produit à une température comprise entre 100 et 200°C en utilisant un dispositif de chauffage du type tunnel à micro-ondes ou à infrarouges, tube de vapeur, autoclave en vapeur vive ou à air chaud, étuve à air ventilé ou à air chaud, four à haute fréquence, ou successivement plusieurs de ces dispositifs, et en le maintenant à cette température pendant une durée comprise entre 1 et 5 heures.

**8.** Procédé selon l'une quelconque des revendications 1 à 7, **caractérisé en ce qu'**il comprend l'incorporation dans la dispersion de fibres de cellulose, le latex ou leur mélange :

- d'un agent tensioactif propre à favoriser la transformation du mélange dispersion de fibres de cellulose/latex, en une mousse et de préférence l'agent tensioactif est choisi parmi les sulfosuccinates et il est utilisé, selon des proportions comprises entre 2 et 6 part en masse pour 100 parts de masse sèche de l'élastomère présente dans le latex ;
- d'un agent propre à stabiliser la mousse, une fois celle-ci formée, et de préférence l'agent propre à stabiliser la mousse est un agent épaississant tel qu'un éther ou un ester de cellulose qui est incorporé dans la dispersion de fibres d'au moins un polymère hydrophile selon des proportions comprises entre 0,5 et 4 parts en masse de la masse sèche de l'élastomère présente dans le latex.

**9.** Procédé selon l'une quelconque des revendications 1 à 8, **caractérisé en ce que** :

- le rapport entre la masse sèche des fibres de polymère hydrophile et la masse sèche de l'élastomère est d'environ 0,5,
- la dispersion de fibres de cellulose présente une concentration en fibres comprise entre 8 et 15 %,
- le latex a une teneur en élastomère sec d'environ 55 %,
- le rapport entre la masse de matière sèche et la masse d'eau présentes dans le mélange est de l'ordre de 0,3.

**10.** Procédé selon l'une quelconque des revendications 1 à 9, **caractérisé en ce qu'**il comprend l'incorporation d'un agent de réticulation au cours de la préparation du mélange fibres de polymère hydrophile/élastomère, notamment aux étapes a), b), d), f) ou g), et de préférence l'agent de réticulation est incorporé dans des proportions comprises entre 0,05 et 0,5 part pour 100 parts de l'élastomère présent dans le latex.

**11.** Matériau alvéolaire comprenant un mélange de fibres de polymère hydrophile et d'au moins un élastomère, susceptible d'être obtenu par le procédé selon l'une quelconque des revendications 1 à 10, dont la structure alvéolaire est formée par des alvéoles dont la taille est comprise entre 0,2 $\mu$m et 10 mm, au moins 1 % des alvéoles, en volume par rapport au volume alvéolaire total, ayant une taille comprise entre 0,2 $\mu$m et 10 $\mu$m.

**12.** Matériau alvéolaire selon la revendication 11, dont au moins 10 % des alvéoles, en volume par rapport au volume alvéolaire total, ont une taille comprise entre 10 $\mu$m et 50 $\mu$m.

**13.** Matériau alvéolaire selon la revendication 11 ou 12, **caractérisé en ce que** le polymère hydrophile est de la cellulose.

**14.** Matériau alvéolaire selon l'une quelconque des revendications 11 à 13, **caractérisé en ce qu'**il présente une ou plusieurs des propriétés suivantes :

- une densité comprise entre 0,01 et 0,1, préférentiellement entre 0,02 et 0,06, encore plus préférentiellement comprise entre 0,03 et 0,05,
- une capacité d'absorption d'eau au moins égale à 800 %, préférentiellement comprise entre 1 000 et 1 600 %,
- une capacité de rétention d'eau après un essorage manuel inférieure à 100 %,
- un taux d'essorage inférieur à 90 %,
- une capacité d'essuyage supérieure ou égale à 65 %, avantageusement supérieure ou égale à 70 %,
- une résistance à la traction au moins égale à 0,1 MPa.

**15.** Matériau alvéolaire selon l'une quelconque des revendications 11 à 14, **caractérisé en ce que** l'élastomère est sélectionné parmi les caoutchoucs polybutadiène, les copolymères de butadiène-styrène, les copolymères butadiène-acrylonitrile, les caoutchoucs nitrile, les caoutchoucs nitrile-butadiène, les copolymères et terpolymères d'éthylène et de propylène, les copolymères séquencés styrène-butadiène, ou styrène-isoprène, les copolymères séquencés styrène-éthylène-butylène-styrène, les élastomères thermoplastiques dérivés des polyoléfines, les copolymères d'octène et d'éthylène, les copolymères d'éthylacrylate, les terpolymères d'acrylate, d'éthylène et d'acide acrylique, les terpolymères d'acrylate, d'acrylonitrile et de styrène, les polychloroprènes, les polyéthylènes chlorés, et leurs mélanges.

16. Matériau alvéolaire selon l'une quelconque des revendications 11 à 15, **caractérisé en ce qu'**il comprend des fibres synthétiques choisies parmi : les fibres de polyamide, les fibres de polyester, les fibres de polyéthylène, la fibre de polypropylène, les fibres de polyacrylonitrile et les fibres d'alcool polyvinylique.

17. Matériau alvéolaire selon la revendication 16, **caractérisé en ce que** les fibres synthétiques représentent au plus 20 % et, de préférence entre 5 et 15 % en masse, de la masse totale des fibres présentes.

18. Matériau alvéolaire selon l'une quelconque des revendications 11 à 17, **caractérisé en ce qu'**il comprend jusqu'à 35 parties en masse, pour 100 parties en masse de l'élastomère, d'un ou de plusieurs polymères propres à servir d'agents d'interface entre les fibres et l'élastomère et choisis parmi les alcools polyvinyliques, les résines mélamine-formaldéhyde, les colles vinyliques et les polyuréthannes.

19. Matériau alvéolaire selon l'une quelconque des revendications 11 à 18, **caractérisé en ce que** le rapport entre la masse totale des fibres et la masse d'élastomère présentes dans ce matériau est compris entre 2 et 0,2 et, de préférence, entre 1,5 et 0,3.

20. Article comprenant un matériau alvéolaire selon l'une quelconque des revendications 11 à 19, qui est sélectionné parmi : une éponge, un article de ménage, un article d'hygiène corporelle tel que : une éponge destinée au bain ; une éponge de soin ou de démaquillage ; une couche pour bébés et un article d'hygiène féminine ; un pansement ; un article destiné à absorber la sueur.

**Patentansprüche**

1. Verfahren zur Herstellung eines Wabenmaterials, das ein Gemisch aus Fasern aus hydrophilem Polymer sowie aus wenigstens einem Elastomer umfasst, **dadurch gekennzeichnet, dass** es umfasst:

a) die Herstellung einer mit $A_1$ bezeichneten wässrigen Dispersion von Fasern aus wenigstens einem hydrophilen Polymer, vorzugsweise aus Zellulose,
b) die Herstellung einer mit $B_1$ bezeichneten Zusammensetzung aus wenigstens einem Elastomer in Form eines Latex,
c) die Zugabe eines Mittels zur Koagulation des Latex in der Dispersion $A_1$, wobei dieser Schritt c) gleichzeitig mit Schritt a) durchgeführt werden kann,
d) das Mischen der bei Schritt c) erhaltenen Dispersion $A_1$ und der Zusammensetzung $B_1$, wobei das Mischen die Koagulation des Latex bewirkt,
e) das Entfernen des Überschusses an bei Schritt c) eingebrachten Koagulationsmittel,
f) optional die Herstellung einer mit $A_2$ bezeichneten wässrigen Dispersion von Fasern aus wenigstens einem hydrophilen Polymer, wobei die Dispersion $A_2$ mit der wässrigen Dispersion $A_1$ identisch oder von dieser verschieden ist, und das Zusetzen dieser wässrigen Dispersion $A_2$ zu dem am Ende des Schrittes e) erhaltenen Gemisch,
g) die Herstellung einer mit $B_2$ bezeichneten Zusammensetzung aus wenigstens einem Elastomer in Form eines Latex, wobei die Zusammensetzung $B_2$ mit der Zusammensetzung $B_1$ identisch oder von dieser verschieden ist, und das Zusetzen dieser Zusammensetzung $B_2$ zu dem bei Schritt e) oder bei Schritt f) erhaltenen Gemisch, wenn ein Schritt f) durchgeführt wird,
h) das Einleiten eines Gases in das Gemisch - Dispersion von Fasern aus wenigstens einem hydrophilen Polymer/Latex/koagulierter Latex - erhalten bei Schritt g), oder das Schlagen des Gemischs, um dem Gemisch eine Wabenstruktur zu verleihen,
i) das Informbringen, insbesondere durch Extrudieren oder durch Formen, des bei Schritt h) erhaltenen Gemischs,
j) das Erhitzen des bei Schritt i) erhaltenen Produktes für die Koagulation und die Vernetzung des Produktes, und
k) das Trocknen des bei Schritt j) erhaltenen Produktes, wobei die Schritte j) und k) in einem einzigen Schritt und in einer einzigen Heizvorrichtung durchgeführt werden können.

**2.** Verfahren nach Anspruch 1, **dadurch gekennzeichnet, dass** die Fasern aus wenigstens einem hydrophilen Polymer einem Fibrillieren unterzogen oder UV-Strahlung ausgesetzt worden sind.

**3.** Verfahren nach Anspruch 1 oder 2, **dadurch gekennzeichnet, dass** bei Schritt c) das Koagulationsmittel eine Säure ist, die der Dispersion $A_1$ in einer Menge zugegeben wird, welche ermöglicht, einen pH-Wert zwischen 1 und 4 zu erhalten, und dass der Schritt e) ein Schritt zum Neutralisieren des am Ende des Schrittes d) erhaltenen Gemischs ist.

**4.** Verfahren nach einem der Ansprüche 1 bis 3, **dadurch gekennzeichnet, dass** $B_1$ 10 bis 80 Gew.-% bezogen auf das Gesamtgewicht des eingebrachten Latex ausmacht und $B_2$ 20 bis 90 Gew.-% bezogen auf das Gesamtgewicht des eingebrachten Latex ausmacht.

**5.** Verfahren nach einem der Ansprüche 1 bis 4, **dadurch gekennzeichnet, dass** das bei Schritt h) eingeleitete Gas, das vorzugsweise aus Luft besteht, in das Gemisch Dispersion von Fasern aus wenigstens einem hydrophilen Polymer/Latex/koagulierter Latex eingebracht wird, indem dieses Gemisch für einige Minuten einem mechanischen Rühren unterzogen wird.

**6.** Verfahren nach einem der Ansprüche 1 bis 5, **dadurch gekennzeichnet, dass** Schritt j) durchgeführt wird, indem das bei Schritt h) erhaltene Produkt bei einer Temperatur von wenigstens gleich 25 °C in einem Mikrowellen- oder Infrarot-Tunnel, einem Dampfrohr, einem Frischdampf- oder Heißluftautoklaven, einem Trockenofen mit ventilierter Luft oder Heißluft, einem Hochfrequenzofen angeordnet wird und dieses Produkt für eine Dauer zwischen 1 und 5 Stunden gehalten wird.

**7.** Verfahren nach einem der Ansprüche 1 bis 6, **dadurch gekennzeichnet, dass** bei Schritt k) das Produkt einem Erhitzungsvorgang unterzogen wird, indem das Produkt einer Temperatur im Bereich zwischen 100 und 200 °C unter Verwendung einer Heizvorrichtung vom Typ Mikrowellen- oder Infrarot-Tunnel, Dampfrohr, Frischdampf- oder Heißluftautoklaven, Trockenofen mit ventilierter Luft oder Heißluft, Hochfrequenzofen oder nacheinander mehreren dieser Vorrichtungen ausgesetzt wird und es für eine Dauer zwischen 1 und 5 Stunden auf dieser Temperatur gehalten wird.

**8.** Verfahren nach einem der Ansprüche 1 bis 7, **dadurch gekennzeichnet, dass** es das Zusetzen zu der Zellulosefaser-Dispersion, dem Latex oder ihrem Gemisch:

- eines Tensids umfasst, das geeignet ist, die Umwandlung des Gemischs Zellulosefaser-Dispersion/Latex in einen Schaum zu begünstigen, und vorzugsweise das Tensid aus den Sulfosuccinaten ausgewählt ist und entsprechend Verhältnissen zwischen 2 und 6 Masseanteilen auf 100 Trockenmasseanteilen des in dem Latex vorhandenen Elastomers verwendet wird,
- eines Mittels umfasst, das geeignet ist, den Schaum zu stabilisieren, sobald dieser gebildet ist, und vorzugsweise das Mittel, welches geeignet ist, den Schaum zu stabilisieren, ein Verdickungsmittel, wie ein Zelluloseether- oder -ester ist, das zu der Dispersion von Fasern aus wenigstens einem hydrophilen Polymer entsprechend Verhältnissen zwischen 0,5 und 4 Masseanteilen der Trockenmasse des in dem Latex vorhandenen Elastomers zugesetzt wird.

**9.** Verfahren nach einem der Ansprüche 1 bis 8, **dadurch gekennzeichnet, dass**:

- das Verhältnis zwischen der Trockenmasse der Fasern aus hydrophilem Polymer und der Trockenmasse des Elastomers etwa 0,5 beträgt,
- die Zellulosefaser-Dispersion eine Faserkonzentration zwischen 8 und 15 % aufweist,
- der Latex einen Trockenelastomergehalt von etwa 55 % aufweist,
- das Verhältnis zwischen der Masse an Trockensubstanz und der Masse an Wasser, die in dem Gemisch vorhanden sind, in der Größenordnung von 0,3 liegt.

**10.** Verfahren nach einem der Ansprüche 1 bis 9, **dadurch gekennzeichnet, dass** es das Zusetzen eines Vernetzungsmittels im Laufe der Herstellung des Gemischs Fasern aus hydrophilem Polymer/Elastomer umfasst, insbesondere bei den Schritten a), b), d), f) oder g), und vorzugsweise das Vernetzungsmittel in Verhältnissen zwischen 0,05 und 0,5 Anteil auf 100 Anteile des in dem Latex vorhandenen Elastomers zugesetzt wird.

**11.** Wabenmaterial, umfassend ein Gemisch aus Fasern aus hydrophilem Polymer sowie aus wenigstens einem Elastomer, das geeignet ist, durch das Verfahren nach einem der Ansprüche 1 bis 10 erhalten zu werden, dessen Wabenstruktur durch Zellen, deren Größe zwischen 0,2 $\mu$m und 10 mm beträgt, gebildet ist, wobei wenigstens 1 Vol.-% der Zellen bezogen auf das Gesamtwabenvolumen eine Größe zwischen 0,2 $\mu$m und 10 $\mu$m hat.

**12.** Wabenmaterial nach Anspruch 11, wobei wenigstens 10 Vol.-% der Zellen bezogen auf das Gesamtwabenvolumen eine Größe zwischen 10 $\mu$m und 50 $\mu$m hat.

**13.** Wabenmaterial nach Anspruch 11 oder 12, **dadurch gekennzeichnet, dass** das hydrophile Polymer Zellulose ist.

**14.** Wabenmaterial nach einem der Ansprüche 11 bis 13, **dadurch gekennzeichnet, dass** es eine oder mehrere der folgenden Eigenschaften aufweist:

- eine Dichte zwischen 0,01 und 0,1, vorzugsweise zwischen 0,02 und 0,06, weiterhin bevorzugt zwischen 0,03 und 0,05,
- eine Wasseraufnahmefähigkeit von wenigstens gleich 800 %, vorzugsweise zwischen 1.000 und 1.600 %,
- ein Wasserrückhaltevermögen nach einem manuellen Wringen von weniger als 100 %,
- eine Wringrate von weniger als 90%,
- eine Wischfähigkeit von mehr als oder gleich 65 %, vorteilhafterweise mehr als oder gleich 70 %,
- eine Zugfestigkeit von wenigstens gleich 0,1 MPa.

**15.** Wabenmaterial nach einem der Ansprüche 11 bis 14, **dadurch gekennzeichnet, dass** das Elastomer ausgewählt ist aus den Polybutadien-Kautschuken, den Butadien-Styrol-Copolymeren, den Butadien-Acrylnitril-Copolymeren, den Nitril-Kautschuken, den Nitril-Butadien-Kautschuken, den Ethylen- und Propylen-Copolymeren und - Terpolymeren, den Styrol-Butadien- oder Styrol-Isopren-Blockcopolymeren, den Styrol-Ethylen-Butylen-Styrol-Blockcopolymeren, den von Polyolefinen abgeleiteten thermoplastischen Elastomeren, den Octen- und Ethylen-Copolymeren, den Ethylacrylat-Copolymeren, den Terpolymeren von Acrylat, Ethylen und Acrylsäure, den Terpolymeren von Acrylat, Acrylnitril und Styrol, den Polychloroprenen, den chlorierten Polyethylenen sowie ihren Mischungen.

**16.** Wabenmaterial nach einem der Ansprüche 11 bis 15, **dadurch gekennzeichnet, dass** es synthetische Fasern umfasst, die aus den Polyamidfasern, den Polyesterfasern, den Polyethylenfasern, den Polypropylenfasern, den Polyacrylnitrilfasern und den Polyvinylalkoholfasern ausgewählt sind.

**17.** Wabenmaterial nach Anspruch 16, **dadurch gekennzeichnet, dass** die synthetischen Fasern höchstens 20 % und vorzugsweise zwischen 5 und 15 Masse-% der Gesamtmasse der vorhandenen Fasern ausmachen.

**18.** Wabenmaterial nach einem der Ansprüche 11 bis 17, **dadurch gekennzeichnet, dass** es bis zu 35 Masseanteile auf 100 Masseanteile Elastomer von einem oder mehreren Polymeren umfasst, die geeignet sind, als Schnittstellenmittel zwischen den Fasern und dem Elastomer zu dienen, und die aus den Polyvinylalkoholen, den Melamin-Formaldehyd-Harzen, den Vinylklebstoffen und den Polyurethanen ausgewählt sind.

**19.** Wabenmaterial nach einem der Ansprüche 11 bis 18, **dadurch gekennzeichnet, dass** das Verhältnis zwischen der Gesamtmasse der Fasern und der Elastomermasse, die in diesem Material vorhanden sind, zwischen 2 und 0,2 und vorzugsweise zwischen 1,5 und 0,3 liegt.

**20.** Artikel, umfassend ein Wabenmaterial nach einem der Ansprüche 11 bis 19, der ausgewählt ist aus: einem Schwamm, einem Haushaltsartikel, einem Körperhygieneartikel, wie: einem Badeschwamm, einem Pflege- oder Abschminkschwamm, einer Babywindel und einem Damenhygieneartikel, einem Verband, einem Artikel zum Aufsaugen von Schweiß.

## Claims

**1.** A method for preparing a cellular material comprising a mixture of hydrophilic polymer fibres and at least one elastomer, **characterized in that** it comprises:

a) preparing an aqueous dispersion, denoted $A_1$, of fibres of at least one hydrophilic polymer, preferably of cellulose;
b) preparing a composition, denoted $B_1$, of at least one elastomer in the form of a latex;

c) adding a latex coagulating agent to dispersion $A_1$, this step c) possibly being performed simultaneously with step a);

d) mixing dispersion $A_1$ obtained at step c) and composition $B_1$; said mixing causing coagulation of the latex;

e) removing excess coagulation agent added at step c);

f) optionally, preparing an aqueous dispersion, denoted $A_2$, of fibres of at least one hydrophilic polymer, dispersion $A_2$ being identical to or differing from aqueous dispersion $A_1$, and incorporating this aqueous dispersion $A_2$ in the mixture obtained after step e);

g) preparing a composition, denoted $B_2$, of at least one elastomer in the form of a latex, composition $B_2$ being identical to or differing from composition $B_1$, and incorporating this composition $B_2$ in the mixture obtained at step e) or step f) if step f) is implemented;

h) injecting a gas into the mixture - dispersion of fibres of at least one hydrophilic polymer/latex/coagulated latex - obtained at step g) or beating said mixture to impart a cellular structure to said mixture;

i) forming the mixture obtained at step h), in particular via extrusion or moulding;

j) heating the product obtained at step i) for coagulation and cross-linking of said product; and

k) drying the product obtained at step j), steps j) and k) possibly being performed in a single step and in a single heating device.

2. The method according to claim 1, **characterized in that** said fibres of at least one hydrophilic polymer have been subjected to fibrillation or exposed to ultraviolet radiation.

3. The method according to claim 1 or 2, **characterized in that** at step c) the coagulation agent is an acid added to dispersion $A_1$ in an amount allowing a pH of between 1 and 4 to be obtained, and **in that** step e) is a neutralisation step of the mixture obtained after step d).

4. The method according to any of claims 1 to 3, **characterized in that** $B_1$ represents 10 to 80 % by weight relative to the total weight of latex used, and $B_2$ represents 20 to 90 % by weight relative to the total weight of latex used.

5. The method according to any of claims 1 to 4, **characterized in that** the gas injected at step h) preferably composed of air is added to the mixture, of the dispersion of fibres of at least one hydrophilic polymer/latex/coagulated latex, by subjecting this mixture for a few minutes to mechanical agitation.

6. The method according to any of claims 1 to 5, **characterized in that** step j) is performed by placing and maintaining the product obtained at step h) at a temperature of at least 25°C in a microwave or infrared tunnel, a steam tube, live steam or hot air autoclave, ventilated-air or hot-air oven, high frequency oven, for a time of between 1 and 5 hours.

7. The method according to any of claims 1 to 6, **characterized in that** at step k) the product undergoes a heating operation by subjecting said product to a temperature of between 100 and 200 °C using a heating device of microwave or infrared tunnel type, or a steam tube, live steam or hot air autoclave, ventilated-air or hot-air oven, high frequency oven, or successively several of these devices, holding the product at this temperature for a time of between 1 and 5 hours.

8. The method according to any of claims 1 to 7, **characterized in that** it comprises the incorporation in the cellulose fibre dispersion, the latex or mixture thereof:

   - of a surfactant able to promote conversion of the cellulose fibre dispersion/latex mixture to a foam, and preferably the surfactant is selected from among sulfosuccinates and is used in proportions of between 2 and 6 parts by weight per 100 parts by dry weight of the elastomer contained in the latex;
   - an agent able to stabilise the foam once it is formed, and preferably the agent able to stabilise the foam is a thickening agent such as a cellulose ether or ester that is incorporated in the dispersion of fibres of at least one hydrophilic polymer in proportions of between 0.5 and 4 parts by weight of the dry weight of the elastomer contained in the latex.

9. The method according to any of claims 1 to 8, **characterized in that**:

   - the ratio between the dry weight of the hydrophilic polymer fibres and the dry weight of the elastomer is about 0.5;
   - the dispersion of cellulose fibres has a fibre concentration of between 8 and 15 %;
   - the latex has a dry elastomer content of about 55 %;
   - the ratio between the weight of dry matter and weight of water contained in the mixture is of the order of 0.3.

**10.** The method according to any of claims 1 to 9, **characterized in that** it comprises the incorporation of a cross-linking agent during preparation of the hydrophilic polymer fibre/elastomer mixture, in particular at steps a), b), d), f) or g) and preferably the cross-linking agent is incorporated in proportions of between 0.05 and 0.5 parts per 100 parts of elastomer contained in the latex.

**11.** A cellular material comprising a mixture of hydrophilic polymer fibres and at least one elastomer, able to be obtained with the method according to any of claims 1 to 10, the cellular structure of which is formed by cells having a size of between 0.2 $\mu$m and 10 mm, at least 1 % of the cells in volume relative to the total cell volume having a size of between 0.2 $\mu$m and 10 $\mu$m.

**12.** The cellular material according to claim 11, at least 10 % of the cells in volume relative to the total cell volume having a size of between 10 $\mu$m and 50 $\mu$m.

**13.** The cellular material according to claim 11 or 12, **characterized in that** the hydrophilic polymer is cellulose.

**14.** The cellular material according to an of claims 11 to 13, **characterized in that** it has one or more of the following properties:

- density of between 0.01 and 0.1, preferably between 0.02 and 0.06, more preferably between 0.03 and 0.05;
- water absorbing capacity of at least 800 %, preferably between 1000 and 1600 %;
- water retaining capacity after manual wringing of less than 100 %;
- spinning rate of less than 90 %;
- wiping capacity of 65 % or higher, advantageously 70 % or higher;
- tensile strength of at least 0.1 MPa.

**15.** The cellular material according to any of claims 11 to 14, **characterized in that** the elastomer is selected from among polybutadiene rubbers, butadiene-styrene copolymers, butadiene-acrylonitrile copolymers, nitrile rubbers, nitrile-butadiene rubbers, copolymers and terpolymers of ethylene and propylene, styrene-butadiene or styreneisoprene block copolymers, styrene-ethylene-butylene-styrene block copolymers, thermoplastic elastomers derived from polyolefins, octene and ethylene copolymers, ethylacrylate copolymers, terpolymers of acrylate, ethylene and acrylic acid, terpolymers of acrylate, acrylonitrile and styrene, polychloroprenes, chlorinated polyethylenes, and mixtures thereof.

**16.** The cellular material according to any of claims 11 to 15, **characterized in that** it comprises synthetic fibres selected from among: polyamide fibres, polyester fibres, polyethylene fibres, polypropylene fibres, polyacrylonitrile fibres and polyvinyl alcohol fibres.

**17.** The cellular material according to claim 16, **characterized in that** the synthetic fibres represent no more than 20 %, and preferably between 5 and 15 %, by weight of the total weight of total fibres.

**18.** The cellular material according to any of claims 11 to 17, **characterized in that** it comprises up to 35 parts by weight, per 100 parts by weight of elastomer, of one of more polymers able to act as interface agents between the fibres and the elastomer and selected from among polyvinyl alcohols, melamine-formaldehyde resins, vinyl glues and polyurethanes.

**19.** The cellular material according to any of claims 11 to 18, **characterized in that** the ratio between the total weight of fibres and weight of elastomer contained in this material is between 2 and 0.2, and preferably between 1.5 and 0.3.

**20.** An article comprising a cellular material according to any of claims 11 to 19, that is selected from among: a sponge, household product, body care item such as: bath sponge; make-up removal or skin care sponge; infant diaper and female sanitary product; a dressing; sweat-absorbing item.

Mouvement rotatif

Figure 1

Mouvements rectilignes

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4559243 A
- WO 9909877 A

- US 20050276956 A1